# EUROPÄISCHE PATENTANMELDUNG

(11) **EP 0 536 081 A1**
(43) Veröffentlichungstag der Anmeldung: **07.04.1993**
(21) Anmeldenummer: 92810617.8
(22) Anmeldetag: 13.08.1992
(51) Int. Cl.: A61F 9/06

(54) **Lichtschutzfilter mit einem bezüglich seiner optischen Transmission elektrisch steuerbaren Filterelement sowie Abschirmmittel für ein Lichtschutzfilter**

(30) Priorität: 27.08.1991 DE 4128291; 10.01.1992 CH 57/92
(71) Anmelder: OPTREL AG, CH-9630 Wattwil (CH)
(72) Erfinder: Eggenschwiler, André M., CH-8712 Stäfa (CH)
(74) Vertreter: Rottmann, Maximilian R.

(57) **Zusammenfassung**

Bei einem Lichtschutzfilter (3), welches insbesondere in der Schweisstechnik zum Einsatz kommt, werden der/die lichtempfindliche(n) Sensor(en) (5, 6), welche in Wirkverbindung mit einer Transmissionssteuereinheit stehen, durch angebrachte oder angeformte Abschirmmittel (10) derart geschützt, dass nur das vom eigentlichen Arbeitsvorgang (z.B. Schweissen) ausgehende Licht eine Änderung der Transmission des Lichtschutzfilters (2) hervorruft und dass z.B. die Arbeitsplatzbeleuchtung den Lichtschutzfilter nicht beeinflusst.

## Beschreibung

Die vorliegende Erfindung betrifft ein Lichtschutzfilter nach dem Oberbegriff des Patentanspruches 1 sowie ein Abschirmmittel für Lichtschutzfilter nach dem Oberbegriff des Patentanspruchs 22.

Aus der EP 0 091 514 A2 sowie anderen Publikationen sind transmissionsgesteuerte Lichtschutzfilter bekannt. Bei diesen Lichtschutzfiltern werden lichtempfindliche Sensoren, welche das Filterelement in gegensinniger Abhängigkeit des auf den Sensor fallenden, hellen Lichtes steuern, eingesetzt.

Die Praxis zeigt nun, dass diese Sensoren durch unerwünschtes Störlicht, welches nicht vom eigentlichen Schweissvorgang ausgeht, beaufschlagt werden können, so dass das Filterelement sich durch dieses Störlicht in seiner optischen Transmission verändert. Bekannte Störlichtquellen sind z.B. Natriumdampflampen oderandere, ähnliche Lichtquellen, wie sie insbesondere in Fabrikhallen zur Beleuchtung des Arbeitsplatzes eingesetzt werden. Für den Benutzer von solchen Lichtschutzfiltern bedeutet das, dass durch die oben erwähnten Störlichtquellen die lichtempfindlichen Sensoren unerwünschterweise ansprechen können, und somit die Lichtdurchlässigkeit des Lichtschutzfilters nicht in allen erforderlichen Fällen gewährleistet ist.

Aufgabe der Erfindung ist es daher, ein Lichtschutzfilter derart zu gestalten, dass Störlicht weitestgehend von den lichtempfindlichen Sensoren zur Transmissionssteuerung ferngehalten wird.

Diese Aufgabe wird durch die im kennzeichnenden Teil des Patentanspruches 1 beschriebenen Massnahmen gelöst.

Weiterbildungen und besondere Ausführungsformen des erfindungsgemässen Lichtschutzfilters sind in den abhängigen Ansprüchen 2 bis 21 beschrieben.

Eine weitere Aufgabe der Erfindung ist, ein Abschirmmittel zu schaffen, das Störlicht weitestgehend von den lichtempfindlichen Sensoren zur Transmissionssteuerung eines Lichtschutzfilters fernhält.

Diese Aufgabe wird durch die im kennzeichnenden Teil des Patentanspruches 22 beschriebenen Massnahmen gelöst.

Weiterbildungen und besondere Ausführungsformen des erfindungsgemässen Abschirmmittels sind in den abhängigen Ansprüchen 23 bis 26 beschrieben.

Ausführungsbeispiele der Erfindung werden im folgenden anhand der beiliegenden Zeichnungen erläutert. In den Zeichnungen zeigen:
Fig.1 eine perspektivische Ansicht, eines Schweisserschutzhelmes mit eingebautem Lichtschutzfilter;
Fig.2 eine perspektivische Ansicht eines ersten Ausführungsbeispieles eines Abschirmmittels;
Fig.2a einen Querschnitt durch das Abschirmmittel gemäss Fig. 2 entlang der Linie I-I;
Fig.3 eine perspektivische Ansicht eines zweiten Ausführungsbeispieles eines Abschirmmittels;
Fig.4 eine perspektivische Ansicht eines dritten Ausführungsbeispieles eines Abschirmmittels;
Fig.5 eine perspektivische Ansicht eines vierten Ausführungsbeispieles eines Abschirmmittels;
Fig.5a eine erste Variante der lichtdurchlässigen Schlitze, in einem vergrösserten Ausschnitt aus der Fig. 5;
Fig.5b eine zweite Variante der lichtdurchlässigen Schlitze, in einem vergrösserten Ausschnitt aus der Fig.5;
Fig.6 eine perspektivische Ansicht eines fünften Ausführungsbeispieles eines Abschirmmittels;
Fig.7a eine Ansicht eines sechsten Ausführungsbeispieles eines Abschirmmittels im Aufriss;
Fig.7b eine Ansicht des sechsten Ausführungsbeispieles eines Abschirmmittels im Grundriss;
Fig.7c eine Ansicht des sechsten Ausführungsbeispieles eines Abschirmmittels im Seitenriss nach der Schnittlinie A-A;
Fig.8a eine Ansicht eines siebten Ausführungsbeispieles eines Abschirmmittels im Aufriss;
Fig.8b eine Ansicht des siebten Ausführungsbeispieles eines Abschirmmittels im Grundriss; und
Fig.8c eine Ansicht des siebten Ausführungsbeispieles eines Abschirmmittels im Seitenriss nach der Schnittlinie B-B.

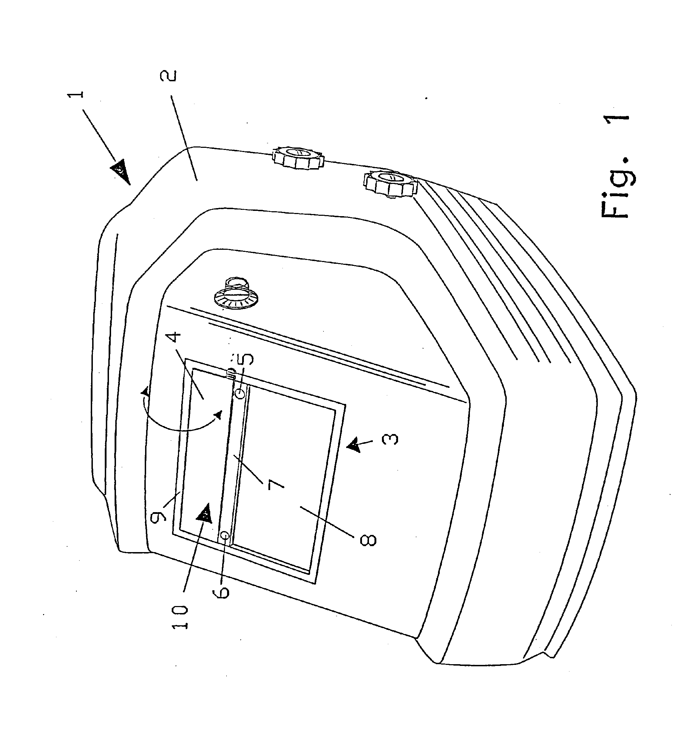

Fig.1 zeigt einen Schweisserschutzhelm 1, in welchem ein transmissionsgesteuertes Lichtschutzfilter 3 zum Einsatz kommt. Im einzelnen weist ein solcher Schweisserschutzhelm 1 das eigentlichen Helmgehäuse 2, eine Lichtschutzkassette 9, ein optoelektrisches Wandlerelement 4, einen Mittelsteg 7, zwei Sensoren 5 und 6 sowie das eigentliche Filterelement 8 auf.

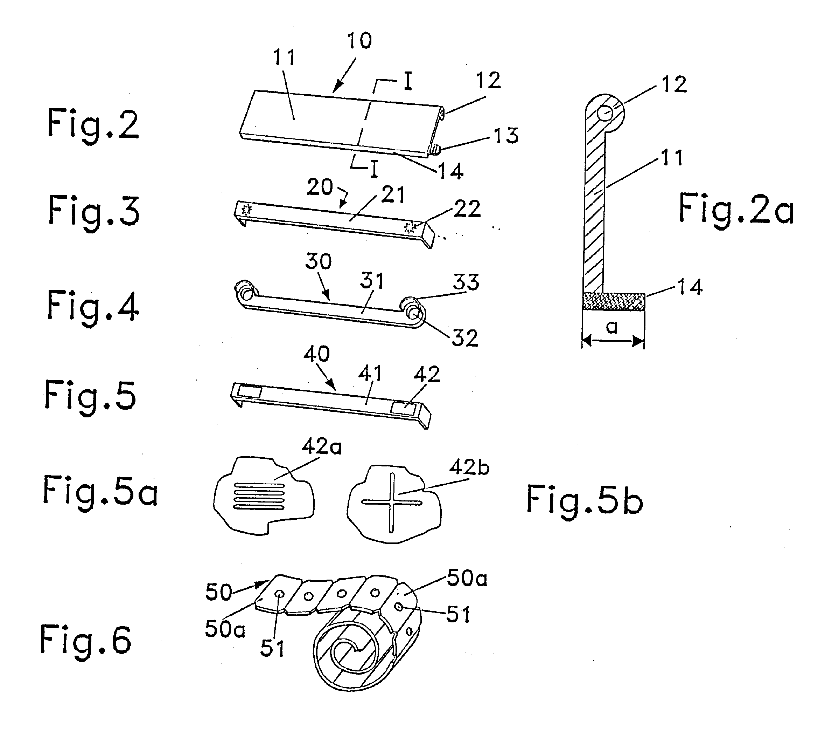

Fig.2 zeigt eine erste Ausführungsform eines Abschirmelementes 10, bestehend aus einer klarsichtabdeckung 11, einem Gelenk 12, welches in diesem Beispiel zur schwenkbaren Befestigung desselben dient, einem Betätigungshebel 13, mit welchem das Abschirmelement hochgeklappt werden kann, sowie einem lichtundurchlässigen Steg 14, welcher bei diesem Beispiel das eigentliche Abschirmelement gegen Störlicht darstellt.

Fig.3 zeigt eine zweite Ausführungsform eines Abschirmelementes 20, bestehend aus einem Bügel 21, an dessen äusseren Enden, im Abstand der auf dem Mittelsteg 7 der Kassette 9 angeordneten, lichtempfindlichen Sensoren 5 und 6, Bohrungen 22 angebracht sind, welche nur unter einem bestimmten Winkel einfallendes Licht auf die Sensoren 5 und 6 passieren lassen. Die Bohrungen 22 können derart ausgebildet sein, dass sie nicht senkrecht zur Oberfläche des Bügels 21, sondern unter einem bestimmten Winkel verlaufen, d.h. dass sie mit der optischen Achse des zugeordneten Sensors einen zweckmässigerweise nach unten gerichteten Winkel einschliessen.

Fig.4 zeigt eine dritte Ausführungsform eines Abschirmelementes 30, bestehend aus einem Bügel 31, an dessen äusseren Enden, im Abstand der auf dem Mittelsteg 7 der kassette 9 angeordneten, lichtempfindlichen Sensoren 5 und 6, Bohrungen 32 angebracht sind; radial dazu sind, mindestens auf eine Seite hin, kappen 33 angeformt, welche allfälliges, unter einem bestimmten Winkel einfallendes Störlicht abschirmen.

Fig.5 zeigt eine vierte Ausführungsform eines Abschirmelementes 40, bestehend aus einem Bügel 41, an dessen äusseren Enden, im Abstand der auf dem Mittelsteg 7 der Kassette 9 angeordeten, lichtempfindlichen Sensoren 5 und 6, lichtdurchlässige Öffnungen 42, angebracht sind, welche allfälliges unter einem bestimmten Winkel einfallendes Störlicht abschirmen.

Die Figuren 5a und 5b zeigen in einervergrösserten Darstellung mögliche Ausführungsformen der lichtdurchlässigen Öffnungen 42. Die in Fig.5a dargestellte Variante weist mehrere horizontale Schlitze 42a auf, während die Variante in Fig.5b sowohl einen vertikalen, wie auch einen horizontalen Schlitz 42b aufweist. Die Ausführungsformen richten sich nach den jeweiligen örtlichen Gegebenheiten, so dass durchaus auch schräge Schlitze (nicht dargestellt) zur Anwendung kommen können.

Fig.6 zeigt eine fünfte Ausführungsform eines Abschirmelementes 50, bestehend aus einem Plättchen 50a, dessen Zentrum eine Bohrung 51 aufweist, welche so über einem oder beiden der Sensoren 5 und 6 angebracht wird, dass das Zentrum der Bohrung mit dem Zentrum des Sensors korrespondiert. Auch hier können die Bohrungen unter einem bestimmten Winkel zur Oberfläche des Plättchens angebracht sein.

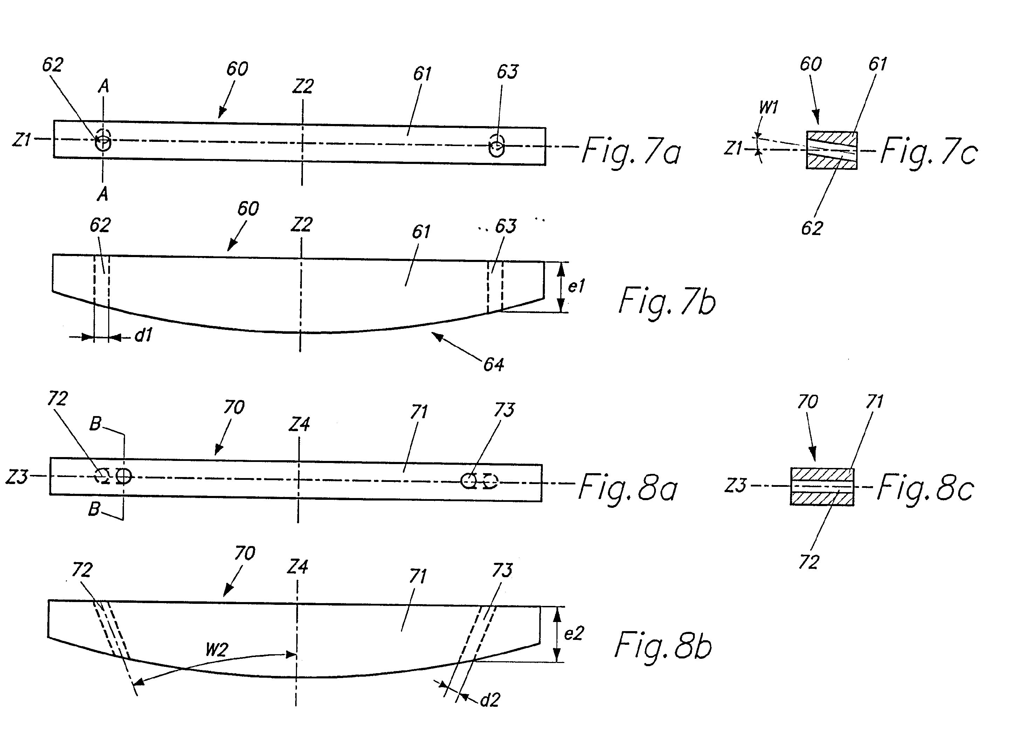

In den Fig. 7a bis 7c ist eine sechste Ausführungsform eines Abschirmelementes ersichtlich. In der Fig.7a ist eine Leiste 61 mit Bohrungen 62 und 63 zu sehen. Mit Z1 ist eine erste Zentralsymmetrieebene bezeichnet, welche in Längsrichtung der Leiste 61 parallel zu der ebenen Oberseite und Unterseite der Leiste 61 verläuft. Mit Z2 ist eine zweite Zentralsymmetrieebene bezeichnet, welche in der Mitte der Leiste 61 parallel zu ebenen Stirnseiten der Leiste 61 verläuft. Die mit 64 bezeichnete Wölbung auf der Vorderseite der Leiste 61 hat keine funktionelle Bedeutung und dient in diesem Ausführungsbeispiel lediglich der Ästhetik, damit sich die Leiste 61 harmonisch in das Gesamtbild des Lichtschutzfilters 3 einfügt.

Die Fig. 7b zeigt die Leiste 61 im Grundriss. Mit d1 ist der Durchmesser der Bohrung 62, 63 bezeichnet, währenddem die Dicke der Leiste im Bereich der Bohrung 62, 63 mit e1 bezeichnet ist. Aus dieser Darstellung ist zu ersehen, dass die Bohrungen 62, 63 parallel zur Zentralsymmetrieebene Z2 ausgeführt sind. In der Fig. 7c ist die Leiste im Schnitt nach der Linie A-A (Fig. 7a) dargestellt. Daraus geht hervor, dass die Achse der Bohrungen 62 und 63 mit derZentralsymmetrieebeneZ1 einen Winkel W1 einschliesst. Dies bewirkt, dass die hinter den Bohrungen 62, 63 der Leiste 61 angeordneten lichtempfindlichen Sensoren 5, 6 im wesentlichen nur mit Licht beaufschlagt werden, welches unter dem Winkel W1 gegenüber der Horizontalen einfällt. Auf diese Weise kann verhindert werden, dass nicht vom Schweissvorgang ausgehendes Licht auf die lichtempfindlichen Sensoren 5, 6 trifft. Durch den Bohrungsdurchmesser d1 sowie die Dicke e1 der Leiste 61 kann bestimmt werden, welcher Lichtstrom auf die hinter den Bohrungen 62, 63 angeordneten lichtempfindlichen Sensoren 5, 6 treffen soll.

In den Fig. 8a bis 8c ist eine siebte Ausführungsform eines Abschirmelementes ersichtlich. In der Fig.8a ist eine Leiste 71 mit Bohrungen 72 und 73 zu sehen. Generell entspricht die Leiste 71 der in den Fig. 7a bis 7c gezeigten Leiste 61. MitZ3 ist eine erste Zentralsymmetrieebene bezeichnet, welche in Längsrichtung der Leiste 71 parallel zu der ebenen Oberseite und Unterseite der Leiste 71 verläuft. Mit Z4 ist eine zweite Zentralsymmetrieebene bezeichnet, welche in der Mitte der Leiste 71 parallel zu den ebenen Stirnseiten der Leiste 71 verläuft.

Die Fig. 8b zeigt die Leiste 71 im Grundriss. Mit d2 ist der Durchmesser der Bohrung 72, 73 bezeichnet, währenddem die Dicke der Leiste 71 im Bereich der Bohrung 72, 73 mit e2 bezeichnet ist. Aus dieser Darstellung ist zu ersehen, dass die Achse der Bohrungen 72, 73 in mit der Zentralsymmetrieebene Z4 einen Winkel W2 einschliesst. Dadurch kann speziell von der Seite einfallendes Störlicht abgeschirmt werden. Wie anhand der Fig. 7b bereits erläutert, kann durch den Bohrungsdurchmesser d2 der Bohrungen 72, 73 sowie die Dicke e2 der Leiste 71 bestimmt werden, welcher Lichtstrom auf die hinter den Bohrungen 72, 73 angeordneten lichtempfindlichen Sensoren 5, 6 treffen soll. In der Fig. 8c ist die Leiste im Schnitt nach der Linie B-B (Fig. 8a) dargestellt. Daraus geht hervor, dass die Achsen der Bohrungen 72 und 73 parallel zur Zentralsymmetrieebene Z3 verlaufen.

Natürlich ist auch eine Kombination der "Schrägstellung" der Bohrungen 62, 63, 72, 73, wie sie anhand der Figurenbeschreibungen 7c sowie 8b erläutert wurden, denkbar.

Die in den Figuren 2 bis 8 dargestellten Abschirmmittel können direkt an der Lichtschutzkassette angeformt sein. Falls aber ein Schweisserschutzhelm bereits in grösseren Stückzahlen, ohne diese Abschirmmittel, verkauft wurde, kann auch die Notwendigkeit bestehen, diese Helme mit solchen Abschirmmitteln nachzurüsten. Die in den Figuren 2 bis 8 aufgeführten Abschirmmittel sind daher so gestaltet, dass sie z.B. einzeln wie auch als Satz erhältlich sind und somit dem Benutzer die Möglichkeit geben, durch Austausch dieser Abschirmmittel das Lichtschutzfilter an unterschiedliche Lichtverhältnisse anzupassen.

## Patentansprüche

1. Lichtschutzfilter, insbesondere für Schweisserschutzhelme (1), Schweisserschutzbrillen oder Schweisserschutzschilder, mit einem bezüglich seiner optischen Transmission elektrisch steuerbaren Filterelement (8), das mit einer Transmissionssteuereinheit in Wirkverbindung steht, welche mindestens einen lichtempfindlichen Sensor (5, 6) umfasst und welche das Filterelement (8) in gegensinniger Abhängigkeit von der Intensität des auf den oder die Sensor(en) (5, 6) fallenden Lichtes auf unterschiedliche Transmissionswerte einstellt, dadurch gekennzeichnet, dass der (die) Sensor(en) (5, 6), welche das Filterelement (8) steuern, mit Abschirmmitteln (10, 20, 30, 40, 50,) versehen ist (sind), welche einfallendes Störlicht auf mindestens einer Seite abschirmen.

2. Lichtschutzfilter nach Anspruch 1, dadurch gekennzeichnet, dass die Abschirmmittel (10) eine Blende miteinem-lichtdurchlässigen Teil (11) und einen zumindest annähernd senkrecht dazu stehenden, lichtundurchlässigen, sich über die Längsausdehnung des lichtdurchlässigen Teils (11) erstrekkenden Steg (14) umfassen, so dass das unter einem bestimmten Winkel einfallende Störlicht abgeschirmt wird.

3. Lichtschutzfilter nach Anspruch 2, dadurch gekennzeichnet, dass das Abschirmmittel (10) mittels eines Scharniers (12) am Lichtschutzfilter (3) schwenkbar befestigt ist und einen Betätigungsbügel (13) aufweist, mittels welchem dieses von einer Wirkstellung in eine inoperative Stellung bewegt werden kann.

4. Lichtschutzfilter nach Anspruch 1, dadurch gekennzeichnet, dass die Abschirmmittel (20) einen Bügel (21) aufweisen, der mit lichtdurchlässigen Bohrungen (22), die in der Lage mit dem/den Sensor(en) (5, 6) korrespondieren, versehen ist, so dass das unter einem bestimmten Winkel einfallende Störlicht abgeshirmt wird.

5. Lichtschutzfilter nach Anspruch 1, dadurch gekennzeichnet, dass die Abschirmmittel (30) einen Bügel (31) aufweisen, der mit lichtdurchlässigen Bohrungen (32), die in der Lage mit dem/den Sensor(en) (5, 6) korrespondieren, versehen sind und an deren äusseren Rand Abschirmkappen (33) angebracht sind, so dass das unter einem bestimmten Winkel einfallende Störlicht abgeschirmt wird.

6. Lichtschutzfilter nach Anspruch 1, dadurch gekennzeichnet, dass die Abschirmmittel (40) einen Bügel (41) aufweisen, der mit lichtdurchlässigen, schrägen, horizontalen und/oder vertikalen öffnungen (42a, 42b) versehen ist, durch die das unter einem bestimmten Winkel einfallende Störlicht abgeschirmt wird.

7. Lichtschutzfilter nach Anspruch 1, dadurch gekennzeichnet, dass die Abschirmmittel (50) mindestens ein Plättchen (50a) aufweisen, das mit einer Bohrung (51) versehen ist, welche so gestaltet ist, dass das unter einem bestimmten Winkel einfallende Störlicht abgeschirmt wird, und welches Plättchen so anbringbar ist, dass das Zentrum der Bohrung(en) (51) mit dem Zentrum des Sensors resp. der Sensoren (5, 6) übereinstimmt.

8. Lichtschutzfilter nach Anspruch 1, dadurch gekennzeichnet, dass die Abschirmmittel (60, 70) aus einer länglichen Leiste (61, 71) bestehen, die mit mindestens einer Bohrung (62, 63, 72, 73) versehen ist, welche in ihrer Lage mit dem/den Sensor(en) (5, 6) korrespondiert.

9. Lichtschutzfilter nach Anspruch 8, dadurch gekennzeichnet, dass die Mittelachse der Bohrung(en) (62, 63, 72, 73) mit mindestens einer Zentralsymmetrieebene (Z1, Z2, Z3, Z4) einen Winkel (W1, W2) einschliesst, derart dass der Einfallswinkel des auf den/die Sensor(en) fallenden Lichtes wählbar ist.

10. Lichtschutzfilter nach Anspruch 8 oder 9, dadurch gekennzeichnet, dass durch das Mass (d1, d2) des Durchmessers der Bohrungen (62, 63, 72, 73) der auf den/die Sensor(en) (5, 6) fallende Lichtstrom bestimmt ist.

11. Lichtschutzfilter nach Anspruch 8 oder 9, dadurch gekennzeichnet, dass durch die Dicke (e1, e2) der Leiste der auf den/die Sensor(en) (5, 6) fallende Lichtstrom bestimmt ist.

12. Lichtschutzfilter nach einem derAnsprüche 1 und 4 bis 11, dadurch gekennzeichnet, dass die Abschirmmittel (20, 30, 40, 50, 60, 70) an der Lichtschutzkassette (3) angeformt sind.

13. Lichtschutzfilter nach einem der Ansprüche 1 bis 11, dadurch gekennzeichnet, dass die Abschirmmittel (10, 20, 30, 40, 50, 60, 70) aufsteckbarsind.

14. Lichtschufilter nach einem der Ansprüche 1 bis 11, dadurch gekennzeichnet, dass die Abschirmmittel (10, 20, 30, 40, 50, 60, 70) anklemmbar sind.

15. Lichtschutzfilter nach einem der Ansprüche 1 bis 11, dadurch gekennzeichnet, dass die Abschirmmittel (10, 20, 30, 40, 50, 60, 70) gegeneinander austauschbar sind.

16. Lichtschutzfilter nach einem der Ansprüche 1 bis 11, dadurch gekennzeichnet, dass die Abschirmmittel (10,20,30,40,50,60,70) auf- oderanklebbar sind.

17. Lichtschutzfilter nach einem der Ansprüche 4 oder 7, dadurch gekennzeichnet, dass die in den Abschirmmitteln (20, 50) vorhandenen Bohrungen (22, 51) einen Winkel zu der Senkrechten des Bügels (21) resp. Plättchens (50) aufweisen, derart dass der Einfallswinkel des Lichtes wählbar ist.

18. Lichtschutzfilter nach Anspruch 6, dadurch gekennzeichnet, dass die in den Abschirmmitteln (40) vorhandenen Öffnungen (42a, 42b) in einem Winkel zur Senkrechten des Bügels (41) ausgeführt sind, derart dass der Einfallswinkel des Lichtes wählbar ist.

19. Lichtschutzfilter nach Ansprüchen 2 und 3, dadurch gekennzeichnet, dass durch das Mass (a) des Steges (14) der Einfallswinkel des Lichtes bestimmt ist.

20. Lichtschutfilter nach einem der Ansprüche 1 bis 11, dadurch gekennzeichnet, dass die Abschirmmittel (10, 20, 30, 40, 50, 60, 70) derart ausgebildet sind, dass die Abschirmung im wesentlichen gegen von oben einfallendes Licht wirksam ist.

21. Lichtschutzfilter nach Anspruch 19, dadurch gekennzeichnet, dass die Abschirmmittel (10, 20, 30, 40, 50, 60, 70) den/ die lichtempfindlichen Sensor(en) (5, 6) gegen einfallendes Störlicht unter einem Winkel von mindestens 90 Grad nach oben abdecken.

22. Abschirmmittel für ein Lichtschutzfilter nach dem Oberbegriff des Patentanspruchs 1, dadurch gekennzeichnet, dass es durch eine längliche Leiste (61, 71) gebildet ist, die mit mindestens einer Bohrung (62, 63, 72, 73) versehen ist, welche in ihrer Lage mit dem/den Sensor(en) (5, 6) korrespondiert.

23. Abschirmmittel nach Anspruch 22, dadurch gekennzeichnet, dass die Achse der Bohrung(en) (62, 63, 72, 73) mit der Zentralsymmetrieebene (Z1, Z2) einen Winkel (W1, W2) einschliesst, derart dass der Einfallswinkel des Lichtes wählbar ist.

24. Abschirmmittel nach Anspruch 22, dadurch gekennzeichnet, dass durch das Mass (d1, d2) des Durchmessers der Bohrungen (62, 63) der auf den/die Sensor(en) (5, 6) fallende Lichtstrom bestimmt ist.

25. Abschirmmittel nach Anspruch 22, dadurch gekennzeichnet, dass durch die Dicke (e1, e2) der Leiste (61, 71) der auf den/die Sensor(en) (5, 6) fallende Lichtstrom bestimmt ist.

26. Abschirmmittel nach einem der Ansprüche 22 bis 25, dadurch gekennzeichnet, dass das Abschirmmittel (60, 70) am Lichtschutzfilter (3) aufsteckbar oder aufklebbar ist.
